# EUROPEAN PATENT APPLICATION

(11) **EP 4 435 090 A1**
(43) Date of publication of application: **25.09.2024**
(21) Application number: 22895764.3
(22) Date of filing: 20.05.2022
(51) Int. Cl.: C12N 1/20, A61K 35/742, A61P 15/08, A23L 33/135, C12R 1/25

(54) **NOVEL LACTOBACILLUS PLANTARUM STRAIN AND USE THEREOF**

(30) Priority: 19.11.2021 KR 20210160740
(71) Applicant: Genome and Company, Seongnam-si, Gyeonggi-do 13486 (KR)
(72) Inventor: KIM, Hyerim, Seongnam-si Gyeonggi-do 13486 (KR); LEE, Suro, Seongnam-si Gyeonggi-do 13486 (KR); YEON, Jaesung, Seongnam-si Gyeonggi-do 13486 (KR); KIM, Hyeyoung, Seongnam-si Gyeonggi-do 13486 (KR); LEE, Donghun, Seongnam-si Gyeonggi-do 13486 (KR)
(74) Representative: Rückerl, Florian
(86) International application number: PCT/KR2022/007256
(87) International publication number: WO 2023/090552

(57) **Abstract**

The present invention relates to a novel *Lactobacillus plantarum* strain. In addition, the present invention relates to a composition for preventing, improving, or treating infertility or subfertility comprising the *Lactobacillus plantarum* strain.

The *Lactobacillus plantarum* strain of the present invention reduces the expression of specific genes in cells constituting the placenta to help implantation of the embryo, increases the number of immune cells in the uterus, and enhances the implantation rate of embryos reduced by immune imbalance. Accordingly, it can be effectively utilized as a composition for the prevention, improvement, or treatment of infertility or subfertility.

## Description

### [Technical Field]

The present invention relates to a novel *Lactobacillus plantarum* strain. In addition, the present invention relates to a composition for preventing, improving, or treating infertility or subfertility comprising the *Lactobacillus plantarum* strain.

### [Background Art]

Infertility or subfertility refers to a condition in which a healthy married man and woman of childbearing age have not yet conceived even after one year despite having a normal sexual life without any contraception. Currently, the number of patients with unexplained infertility or subfertility is gradually increasing, and in the past 10 years, the number of patients has increased by more than 3.7 times in Korea. However, even after undergoing in vitro fertilization, which is a representative treatment for infertility or subfertility, approximately 5% has still difficulty conceiving due to repeated implantation failure.

Accordingly, researches to uncover the causes of infertility or subfertility and developments of drugs to treat it are actively being conducted. In this regard, studies have been published that repeated implantation failure is caused by immunological factors (Jason M Franasiak et al., Fertil Steril. 2017 Jun, 107(6):1279-1283). Research results showing that the microbiomes in the uterus and vagina are important environmental factors in pregnancy and a dominance of *Lactobacillus spp.* may aid in conception and maintenance of pregnancy is attracting attention (Alex Farr et al., PLoS One. 2015 Dec 11, 10(12):e0144181)*.* In addition, Lactin-V medicine based on *Lactobacillus crispatus* is currently undergoing clinical trials and is known to be effective in preventing repetitive bacterial vaginosis and improving the success rate of in vitro fertilization (Craig R Cohen et al., N Engl J Med. 2020 May 14, 382(20):1906-1915)*.*

However, therapies for treating infertility or subfertility from an immunological perspective, or medicines for treating it by using a microbiome immunologically have not yet been developed. There remains a significant need for such treatments in the field to which the present invention belongs.

### [Technical Problem]

The inventors have conducted various researches on strains that can treat infertility or subfertility caused by immunological factors and microbiome imbalance. As a result, they have discovered a novel *Lactobacillus plantarum* strain and have deposited it. The present invention was completed by experimentally demonstrating that the strain regulates the expression of genes that help implantation, increases the number of immune cells in the uterus, and increases the embryo implantation rate.

### [Technical Solution]

The present invention provides a novel *Lactobacillus plantarum* strain.

As used herein, the term "*Lactobacillus plantarum*" refers to a strain deposited by the present applicant at the Korean Collection for Type Cultures (KCTC) of Korea Research Institute of Bioscience and Biotechnology (KRIBB) on August 24, 2021, with Accession No. KCTC 14678BP. In the present invention, "*Lactobacillus plantarum*" may be used interchangeably with "*Lactobacillus plantarum* strain" or "strain". The strain is interpreted to include a strain itself, a culture of the strain, a lysate of the strain, an extract of the strain, and a cytoplasmic fraction obtained by lysing the strain.

The term "culture" refers to a strain itself, extract of the strain obtained by culturing the strain for a certain period on a medium supplemented with nutrient, metabolites of the strain, or the entire medium comprising extra nutrients. It also includes the culture medium from which the strain was removed after culturing it. Additionally, the culture includes the entire medium or a concentrate of the culture medium, and a dried product of the concentrate. Specifically, the medium can be easily selected by a person skilled in the art depending on the purpose among media used for microbial culture.

In the present invention, the *Lactobacillus plantarum* strain can be used for preventing or treating infertility or subfertility. Additionally, the strain can decrease an expression or activity of T cell immunoglobulin and mucin domain-containing protein 3 (TIM3), increase the number of immune cells in the uterus, or increase the embryo implantation rate.

Specifically, the "TIM3" refers to the "T cell immunoglobulin and mucin domain-containing protein 3" gene. Among the cells that make up the embryo, the cells directly involved in implantation are trophoblasts. The trophoblasts surround the blastocyst in the embryo and interact directly with endometrial cells. When the expression of TIMP3 in trophoblasts is reduced, invasion into the endometrial layer increases, thereby leading to successful implantation. Therefore, the *Lactobacillus plantarum* strain of the present invention, which can reduce the expression of TIM3 in trophoblasts, can help with implantation.

Additionally, the immune cells in the uterus may be lymphocytes and/or NK cells. Immune cells such as lymphocytes and NK cells present in the uterus are known to help an embryo implantation when present in sufficient numbers. Therefore, the *Lactobacillus plantarum* strain of the present invention, which can increase the number of immune cells, can help with implantation.

Additionally, the strain of the present invention can increase the implantation rate reduced by immune imbalance to a normal level.

Another aspect of the present invention provides a pharmaceutical composition for preventing or treating infertility or subfertility comprising at least one selected from the group consisting of a *Lactobacillus plantarum* strain deposited under Accession No. KCTC 14678BP, a culture of the strain, a fermentation product of the strain, a lysate of the strain, an extract of the strain, and a cytoplasmic fraction obtained by lysing the strain.

In the pharmaceutical composition according to the present invention, each term is the same as described for the *Lactobacillus plantarum* strain unless specifically mentioned.

The pharmaceutical composition of the present invention comprises the *Lactobacillus plantarum* strain as an active ingredient. In addition, as described above, the *Lactobacillus plantarum* strain is interpreted to include a strain itself, a culture of the strain, a fermentation product of the strain, a lysate of the strain, an extract of the strain, and a cytoplasmic fraction obtained by lysing the strain, and thus the pharmaceutical composition of the present invention comprises the *Lactobacillus plantarum* strain, a culture of the strain, a lysate of the strain, an extract of the strain, or a cytoplasmic fraction obtained by lysing the strain.

As used herein, the terms "infertility" and "subfertility" refer to cases where pregnancy is difficult or impossible to achieve within one year despite normal sexual intercourse without contraception. The infertility or subfertility may be female infertility or female subfertility.

In addition, the infertility or subfertility may be due to one or more causes selected from the group consisting of immunologic aberration, decreased ovarian reserve, ovulatory disorder, implantation failure, tubal injury, tubal blockage, paratubal adhesions, infection, immunologic illness, and endometriosis.

The pharmaceutical composition of the present invention comprises the strain in an effective amount and may be administered to a subject in need of prevention or treatment of infertility or subfertility.

As used herein, the term "administration" or "administering" refers to the physical introduction of the composition to a subject using any various methods and delivery systems known to a person skilled in the art. The route of administration includes, for example, oral, intravaginal, intravenous, intramuscular, subcutaneous, intraperitoneal, spinal or other parenteral routes such as routes through injection or infusion, but are not limited thereto. The administration may be, for example, performed once, multiple times, or over one or more extended periods of time.

The pharmaceutical composition of the present invention may comprise the *Lactobacillus plantarum* strain alone, or may further comprise acceptable carriers, excipients or diluents commonly used for preparing a pharmaceutical composition. The carriers, excipients or diluents that may be comprised in the composition include, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil, but are not limited thereto. These may be used alone, or in a mixture of two or more types. In addition, the pharmaceutical composition may further comprise other accepable additives such as antioxidants, buffers and/or bacteristats, if necessary, and may additionally comprise dispersants, surfactants, binders, lubricants, etc. to be formulated into powders, granules, tablets, liquids, capsules, suspensions, emulsions, syrups, creams, lotions, gels, ointments, aerosols, powder sprays, creams, suppositories, pills, etc.

As an embodiment of the above formulation, the pharmaceutical composition of the present invention may be an intravaginal administration formulation. Specific examples of the intravaginal administration formulation may be one or more selected from the group consisting of tablets, liquids, creams, lotions, gels, ointments, powder sprays, suppositories, and pills, but are not limited thereto.

As used herein, the term "subject" includes a human or any non-human animal. The non-human animal may be, for example, a vertebrate such as a primate, dog, cow, horse, pig, rodent such as mouse, rat, guinea pig, and the like. The term "subject" may be used interchangeably herein with "individual" or "patient".

The effective amount may be a "therapeutically effective amount" or a "prophylactically effective amount". As used herein, the term "therapeutically effective amount" refers to, when a drug or a therapeutic agent is used alone or in combination with other therapeutic agents, any amount capable of having a decrease in the severity of disease, an increase in the frequency and duration of disease symptom-free period, or a prevention of damage or impairment due to disease suffering. As used herein, the term "prophylactically effective amount" refers any amount that inhibits the occurrence or recurrence of infertility or subfertility in a subject. The level of effective amount may be determined depending on factors including severity, age, sex, drug activity, drug sensitivity, administration time, administration route, excretion rate, treatment period, drugs used concurrently, and other factors well known in the medical field.

In addition, the administration of the pharmaceutical composition may vary depending on age, gender, and weight of subject. Specifically, depending on the symptoms of the subject, 0.1 to 100 mg/kg of the composition of the present invention may be administered once to several times daily, or at intervals of several days to several months. Additionally, the dosage may increase or decrease depending on the route of administration, severity of disease, gender, weight, age, etc.

The pharmaceutical composition may be administered in combination with other therapeutic agents. In this case, the pharmaceutical composition of the present invention and the other therapeutic agents may be administered simultaneously, sequentially, or separately. The other therapeutic agents may be a drug such as a compound, protein, or hormone agent that has the effect of preventing, treating, or improving infertility or subfertility, but are not limited thereto. For example, the hormone agent may be progesterone.

Additionally, the pharmaceutical composition according to the present invention may be formulated to be administered simultaneously, sequentially, or separately with other therapeutic agents. For example, the strain and the other therapeutic agents may be administered simultaneously as one formulation or may be administered simultaneously, sequentially, or separately as separate formulations. For simultaneous, sequential, or separate administration, the strain comprised in the pharmaceutical composition of the present invention and the other therapeutic agents may be formulated separately in each container or may be formulated together in the same container. In addition, the effective amount, administration time, administration interval, administration route, treatment period, etc. of the strains comprised in the composition of the present invention and the other therapeutic agents may be the same or different from each other.

Another aspect of the present invention provides a use of preventing or treating infertility or subfertility of at least one selected from the group consisting of a *Lactobacillus plantarum* strain deposited under Accession No. KCTC 14678BP, a culture of the strain, a fermentation product of the strain, a lysate of the strain, an extract of the strain, and a cytoplasmic fraction obtained by lysing the strain.

In the use of prevention or treatment according to the present invention, each term has the same meaning as described for the aforementioned strain or pharmaceutical composition, unless specifically stated otherwise.

Another aspect of the present invention provides a method of preventing or treating infertility or subfertility comprising administering a subject in need thereof at least one selected from the group consisting of a *Lactobacillus plantarum* strain deposited under Accession No. KCTC 14678BP, a culture of the strain, a fermentation product of the strain, a lysate of the strain, an extract of the strain, or a cytoplasmic fraction obtained by lysing the strain.

In the method of prevention or treatment according to the present invention, each term has the same meaning as described for the aforementioned strain or pharmaceutical composition, unless specifically stated otherwise.

In the method of prevention or treatment according to the present invention, the *Lactobacillus plantarum* strain deposited under Accession No. KCTC 14678BP may be administered to a subject simultaneously, sequentially, or separately with other therapeutic agents.

The "simultaneous" administration means that *Lactobacillus plantarum* strain and the other therapeutic agents are administered at once in one formulation, or *Lactobacillus plantarum* strain and the other therapeutic agents are administered at once in separate formulations, in which the route of administration thereof may be different each other. The "sequential" administration refers to a relatively continuous administration of the *Lactobacillus plantarum* strain and the other therapeutic agents, allowing the administration interval to a minimum. The "separate" administration refers to an administration of the *Lactobacillus plantarum* strain and the other therapeutic agents at predetermined time intervals. The administration method of the *Lactobacillus plantarum* strain and the other therapeutic agents may be appropriately selected by a doctor or expert in the art in consideration of the treatment efficacy or side effects of subject.

Another aspect of the present invention provides a food composition for preventing or improving infertility or subfertility comprising at least one selected from the group consisting of a *Lactobacillus plantarum* strain deposited under Accession No. KCTC 14678BP, a culture of the strain, a fermentation product of the strain, a lysate of the strain, an extract of the strain, and a cytoplasmic fraction obtained by lysing the strain.

In the food composition according to the present invention, each term has the same meaning as described for the aforementioned strain or pharmaceutical composition, unless specifically stated otherwise.

The food may be a health functional food. As used herein, the term "health functional food" refers to a food manufactured and processed in the form of tablets, capsules, powders, granules, liquids, pills, etc. using raw materials or ingredients having functions useful to the human body. Herein, "functional" or "functionality" refers to obtaining effects useful for health purposes such as regulating nutrients or physiological actions with respect to the structure and function of the human body.

The type of the food is not limited. For example, it may be commercialized or formulated as yogurt, dairy products, meat, sausage, bread, chocolate, candy, snacks, confectionery, pizza, ramen, noodles, gum, dairy products including ice cream, soup, beverage, tea, drinks, alcoholic beverages, or vitamin complexes, and includes all food products in the conventional sense.

The food composition according to the present invention may be manufactured by a method commonly used in the art, and may be manufactured by adding raw materials and components commonly added in the art. Unlike conventional drugs, the food has an advantage of having no side effects that may occur with long-term use of drugs, and is highly portable. Therefore, the food composition of the present invention can be used as a supplement for enhancing the effect to prevent or improve infertility or subfertility.

The effective amount of *Lactobacillus plantarum* strain comprised in the food composition according to the present invention may be appropriately determined depending on the purpose of use such as prevention, improvement, or therapeutic treatment. Generally, *Lactobacillus plantarum* strain, a culture of the strain, a fermentation product of the strain, a lysate of the strain, an extract of the strain, or a cytoplasmic fraction obtained by lysing the strain may be comprised in an amount of 0.001 to 20 percent by weight (wt%), 0.001 to 15 wt%, or 0.001 to 10 wt%, in the food composition. In the case of health drinks, it may be comprised in an amount of 0.01 to 2 g, specifically 0.02 to 2 g, more specifically 0.3 to 1 g based on 100 mL. However, in the case of long-term ingestion for health and hygiene purposes or health control, it is possible to use in an amount less than the above range. The *Lactobacillus plantarum* strain added to the food composition in the process of preparing it may be appropriately increased or decreased as necessary.

The food composition may be in any one formulation such as pills, tablets, granules, powders, capsules, liquids, and solutions.

The food composition may comprise various sweeteners or natural carbohydrates as additional ingredients like conventional food. The natural carbohydrates may be, for example, monosaccharides such as glucose and fructose, disaccharides such as maltose and sucrose, polysaccharides such as dextrin and cyclodextrin, and sugar alcohols such as xylitol, sorbitol, and erythritol. The sweeteners may be, for example, a natural sweetener such as thaumatin and stevia extract, or a synthetic sweetener such as saccharin and aspartame.

In addition, when the food composition is a beverage composition, there is no limitation on the liquid ingredients if it comprises *Lactobacillus plantarum* strain as an active ingredient in the indicated ratio. Like a conventional beverage, it may comprise various flavoring agents or natural carbohydrates as additional ingredients.

The *Lactobacillus plantarum* strain of the present invention reduces the expression of specific genes in cells constituting the placenta to help implantation of the embryo, increases the number of immune cells in the uterus, and enhances the implantation rate of embryos reduced by immune imbalance. Accordingly, it can be effectively utilized as a composition for the prevention, improvement, or treatment of infertility or subfertility.

### [Brief Description of Figures]

FIG. 1 is a graph showing the effect of reducing the TIM3 gene expression level of the *Lactobacillus plantarum* strain according to the present invention. * means P < 0.05, ** means P < 0.01, and *** means P < 0.001.
FIG. 2 is a schematic diagram of an experiment performed to confirm the effect of increasing the embryo implantation success rate of the *Lactobacillus plantarum* strain according to the present invention.
FIG. 3 is a graph showing the effect of the *Lactobacillus plantarum* strain according to the present invention on increasing uterine immune cells. A refers to the total number of lymphocytes and B refers to the number of uterine NK cells.

### [Examples]

Hereinafter, the present invention will be described in more detail by Examples. However, these examples are only for illustrative purposes, and the scope of the present invention is not limited thereto.

### Example 1. TIM3 expression inhibition effect

When the endometrium is thick and implantation of an embryo occurs, the lower the expression of tissue inhibitors of metalloproteinase (TIMP3) in trophoblasts, the more favorable it is for implantation (Jia-Yu Zhu et al., Rev Obstet Gynecol. 2012 ;5(3-4):e137-43). Therefore, if the *Lactobacillus plantarum* strain, deposited by the applicant with the Korea Research Institute of Bioscience and Biotechnology under the accession number KCTC 14678BP on August 24, 2021, reduces the expression of the TIMP3 gene in the trophoblast HTR-8/SVneo cell line, it proves that it is the strain that can increase the implantation rate and pregnancy rate.

Specifically, HTR-8 cells were seeded at 4 × 10⁵ cells/ml in a 12-well plate the day before the experiment. The next day, after confirming under a microscope that the confluency was 70-80% and that the layer was evenly formed, it was washed with PBS and exchanged for 0.8 ml of serum-free medium. Thereafter, the strain suspension was treated to have a multiplicity of infection (MOI) of 50 or 100 relative to the number of cells. As a positive control, leukemia inhibitory factor (LIF) expressed in endometrial cells, which is an important factor for pregnancy and implantation, was used (Pankaj Suman et al., Fertil Steril. 2013 Feb;99(2):533 -42), and treated so that the final concentration was 50 ng/ml. After 4 hours of LIF treatment, RNA was extracted and the 18S gene and TIMP3 gene were amplified through cDNA synthesis, and the expression level of each gene was calculated relative to the 18S rRNA expression level.

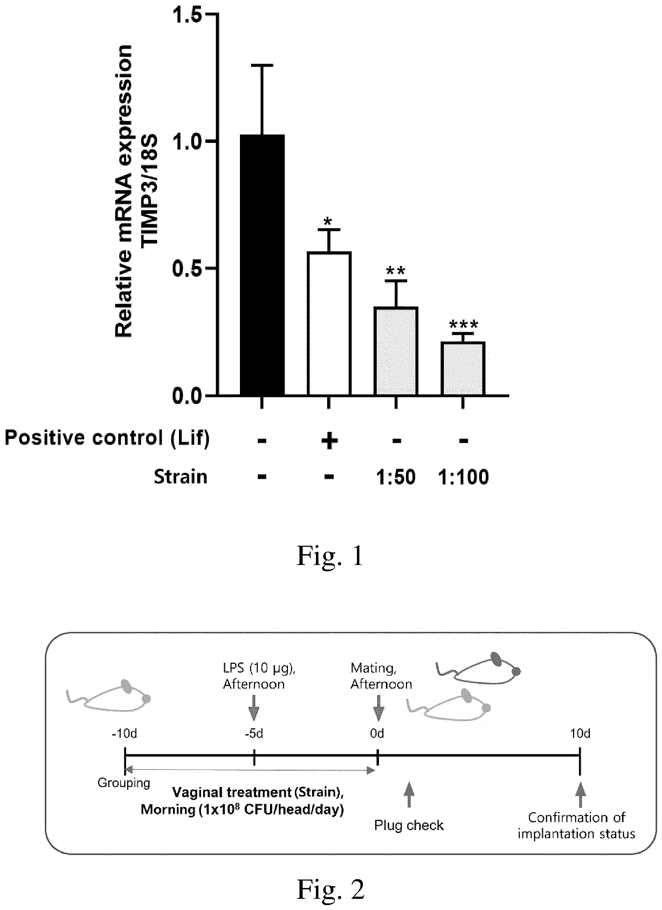

As a result, as can be seen in FIG. 1, when treated with LIF in a positive control group, it was confirmed that the expression level of the TIMP3 gene was significantly reduced compared to the untreated control group. In addition, when the strain was treated with 50 or 100 MOI relative to the number of cells, it was confirmed that the expression level of the TIMP3 gene decreased in a dose-dependent manner, which showed a significant effect of about 2 times more than a positive control group treated with LIF.

From the above results, it shows that the *Lactobacillus plantarum* strain of the present invention can reduce the expression level of the TIM3 gene, and thus helps the implantation process of the embryo, thereby preventing or treating infertility or subfertility.

### Example 2. Effect of increasing implantation success rate

In order to confirm whether the *Lactobacillus plantarum* strain of the present invention, deposited under the accession number KCTC 14678BP, can prevent repetitive implantation failure due to immunological causes and microbiome imbalance, the implantation rate was assessed after administering the *Lactobacillus plantarum* strain to a mouse model of LPS-induced implantation failure. Implantation failure was induced by administering LPS, which is known to cause immune imbalance (Sarah Moustafa et al., Am J Reprod Immunol. 2019 Feb; 81(2): e13082).

Specifically, the control group and experimental group were set as follows:
- Control group: normal mice not treated with strain nor LPS
- Negative control group (LPS treated group): mice treated with LPS to induce implantation failure
- Experimental group (LPS and strain treatment group): mice treated with LPS during treatment of the *Lactobacillus plantarum* strain to induce implantation failure

After randomly separating eight female balb/c mice in each group, PBS for the Control group, PBS for the Negative control group, and 1 × 10⁸ CFU of the strain dissolved in PBS for the Experimental group were intravaginally administered in the morning for 10 days (-10d to 0d). Based on the first day (-10d) of strain treatment, 10 µg of LPS dissolved in PBS was intravaginally administered to the Negative control and Experimental groups on the afternoon of the 5th day (-5d). Afterwards, on the afternoon of the 10th day (0d), mating was conducted for 3 days with a female to male ratio of 2:1. Based on the first day of mating (0d), dissection was performed on the 10th day (10d) to confirm the success or failure of implantation. A schematic diagram of this experiment is shown in FIG. 2.

**Table 1**

| | normal | Negative control (LPS treatment group) | Experimental group (LPS and strain treatment group) |
|---|---|---|---|
| Implantation success rate (%) | 87.5% | 25% | 87.5% |
| Average number of embryos implanted | 7 | 5 | 7.57 |

As a result, as can be seen in Table 1, the implantation success rate of the Control group was 87.5%, but that of the Negative control group treated with LPS was 25%, showing a decrease of about 62.5% compared to the Control group. On the other hand, it was confirmed that the Experimental group treated the strain showed an implantation success rate of 87.5%, which is the same level as the Control group, despite LPS treatment. From the above results, it shows that the *Lactobacillus plantarum* strain of the present invention has the effect of increasing the implantation rate of embryos reduced by immune imbalance, and thus can be usefully used to prevent or treat infertility or subfertility.

### Example 3. Effect of increasing the number of uterine immune cells

It is known that enough immune cells present in the uterus help the implantation of an embryo. In particular, lymphocytes and NK cells in the uterus (uterine NK; uNK) play an important role in the maturation of decidual NK cells, which are essential for the implantation process. In the absence of uNK, even if the embryo is implanted, it may fall out afterwards (Dorothy K Sojka et al., Front Immunol. 2019 May 1;10:960*;* Nabila Jabrane-Ferrat et al., Immunology. 2014 Apr; 141(4): 490-497)*.* Accordingly, it was assessed how the *Lactobacillus plantarum* strain of the present invention, deposited under the accession number KCTC 14678BP, affects uNK and related immunity in the uterus.

Specifically, the strain was administered to a mouse model in which implantation failure was induced by the method according to Example 2. The control and experimental groups were set as follows:
- Control group: normal mice not treated with strain nor LPS
- Negative control group (LPS treated group): mice treated with LPS to induce implantation failure
- Experimental group (LPS and strain treatment group): mice treated with LPS during treatment of the *Lactobacillus plantarum* strain to induce implantation failure

After completing the treatment of each substance, the uterine tissue of the mouse was extracted and chopped, and the tissue was isolated using separation buffer and immune cells were isolated using FBS. The isolated immune cells (1×10⁶ cells/ml) were transferred to a 1.5 ml tube and Fc block was attached. NK cell antibodies (CD45⁺, CD3⁻, CD335⁺, CD49b⁺) were attached to the immune cells. The number of total lymphocytes and uNK cells was analyzed by flow cytometry.

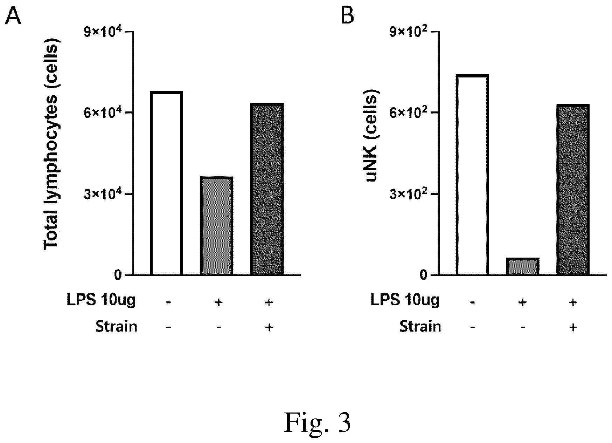

As a result, as can be seen in A in FIG. 3, it was confirmed that the Negative control group treated with LPS showed the total number of lymphocytes significantly reduced compared to the Control group. On the other hand, in the case of the Experimental group treated with LPS and the strain, it was confirmed that the total number of lymphocytes reduced by LPS increased and recovered to a level similar to that of the Control group.

In addition, as can be seen in B in FIG. 3, it was confirmed that the Negative control group treated with LPS showed the number of uNK significantly reduced compared to the Control group. On the other hand, in the case of the Experimental group treated with LPS and the strain, it was confirmed that the number of uNK reduced by LPS increased and recovered to a level similar to that of the Control group.

From the above results, it shows that the *Lactobacillus plantarum* strain of the present invention has the effect of increasing the number of immune cells in the uterus that help implantation, and thus can be usefully used to prevent or treat infertility or subfertility

## Claims

1. *A Lactobacillus plantarum* strain deposited under Accession No. KCTC 14678BP.

2. The *Lactobacillus plantarum* strain of claim 1, wherein the strain is used for preventing or treating infertility or subfertility.

3. The *Lactobacillus plantarum* strain of claim 1, wherein the strain decreases an expression or activity of T cell immunoglobulin and mucin domain-containing protein 3 (TIM3), increases the number of immune cells in a uterus, or increases an implantation rate of embryo.

4. A pharmaceutical composition for preventing or treating infertility or subfertility comprising at least one selected from the group consisting of a *Lactobacillus plantarum* strain deposited under Accession No. KCTC 14678BP, a culture of the strain, a fermentation product of the strain, a lysate of the strain, an extract of the strain, and a cytoplasmic fraction obtained by lysing the strain.

5. The pharmaceutical composition of claim 4, wherein the infertility or subfertility is a female infertility or female subfertility.

6. The pharmaceutical composition of claim 4, wherein the infertility or subfertility is caused by at least one selected from the group consisting of immunologic aberration, decreased ovarian reserve, ovulatory disorder, implantation failure, tubal injury, tubal blockage, paratubal adhesion, infection, immunologic illness, and endometriosis.

7. The pharmaceutical composition of claim 4, wherein the pharmaceutical composition is administered by an intravaginal route.

8. The pharmaceutical composition of claim 4, wherein the pharmaceutical composition is formulated into tablets, liquids, creams, lotions, gels, ointments, powder sprays, suppositories, or pills.

9. The pharmaceutical composition of claim 4, wherein the pharmaceutical composition is administered in combination with progesterone.

10. A food composition for preventing or improving infertility or subfertility comprising at least one selected from the group consisting of a *Lactobacillus plantarum* strain deposited under Accession No. KCTC 14678BP, a culture of the strain, a fermentation product of the strain, a lysate of the strain, an extract of the strain, and a cytoplasmic fraction obtained by lysing the strain.

11. The food composition of claim 10, wherein the infertility or subfertility is a female infertility or female subfertility.

12. The food composition of claim 10, wherein the infertility or subfertility is caused by at least one selected from the group consisting of immunologic aberration, decreased ovarian reserve, ovulatory disorder, implantation failure, tubal injury, tubal blockage, paratubal adhesion, infection, immunologic illness, and endometriosis.

13. The food composition of claim 10, wherein the food composition is ingested in combination with progesterone.

14. A use of preventing or treating infertility or subfertility of at least one selected from the group consisting of a *Lactobacillus plantarum* strain deposited under Accession No. KCTC 14678BP, a culture of the strain, a fermentation product of the strain, a lysate of the strain, an extract of the strain, and a cytoplasmic fraction obtained by lysing the strain.

15. A method of preventing or treating infertility or subfertility comprising administering a subject in need thereof at least one selected from the group consisting of a *Lactobacillus plantarum* strain deposited under Accession No. KCTC 14678BP, a culture of the strain, a fermentation product of the strain, a lysate of the strain, an extract of the strain, and a cytoplasmic fraction obtained by lysing the strain.
